# FASCICULE DE BREVET EUROPEEN

(11) **EP 1 720 849 B1**
(45) Date de publication et mention de la délivrance du brevet: **07.05.2008**
(21) Numéro de dépôt: 05717419.5
(22) Date de dépôt: 14.01.2005
(51) Int. Cl.: C07D 309/38, A61K 31/366, A61P 35/00, C07C 47/273

(54) **DERIVES DE PYRANONE UTILES POUR LE TRAITEMENT DU CANCER**
ZUR BEHANDLUNG VON KREBS GEEIGNETE PYRANONDERIVATE
PYRANONE DERIVATIVES USEFUL FOR TREATING CANCER

(30) Priorité: 14.01.2004 FR 0400298
(43) Date de publication de la demande: 15.11.2006
(73) Titulaire: CENTRE NATIONAL DE LA RECHERCHE SCIENTIFIQUE (CNRS) Etablissement Public, 75016 Paris (FR)
(72) Inventeur: BAKALA, Joanna, F-75012 Paris (FR); HERLEM, Denyse, F-78000 Versailles (FR); BENECHIE, Emile, F-91190 Gif S/Yvette (FR); BIGNON, Jérôme, F-91530 Le Val Saint Germain (FR); KHUONG-HUU, Françoise, F-75015 Paris (FR); POTIER, Pierre, DÉCÉDÉ (FR)
(74) Mandataire: Ahner, Francis
(86) Numéro de dépôt international: PCT/FR2005/000084
(87) Numéro de publication internationale: WO 2005/073211

(56) Documents cités:
- US-A- 5 093 505

## Description

La présente invention a pour objet des dérivés de pyranone de formule générale (I), leur procédé de préparation et leur application thérapeutique, notamment pour le traitement du cancer. La présente invention a également pour objet les composés intermédiaires de formule générale (IV).

Les maladies tumorales, qui touchent dix millions de personnes à travers le monde, sont après les maladies cardiovasculaires, les affections les plus meurtrières. Les efforts déployés ces dernières années dans différents domaines de recherche, ont conduit à une amélioration importante de la thérapie du cancer. Les progrès de l'oncologie médicale sont dus en grande partie à la mise sur le marché de nouveaux médicaments cytotoxiques (cisplatine, taxoïdes, irinotécam, topotécam...). Aujourd'hui, qu'elle soit associée ou non à la radiothérapie et à la chirurgie, la chimiothérapie reste le traitement prédominant dans de nombreux cancers. Ainsi, les médicaments cytotoxiques peuvent être administrés avant une intervention chirurgicale ou une radiothérapie afin de réduire la taille de la tumeur. Ils sont également très souvent nécessaires après la chirurgie ou la radiothérapie afin d'éliminer toutes les cellules cancéreuses qui auraient pu résister à ces traitements.

Pourtant, en dépit de l'introduction régulière de nouveaux médicaments, l'approche chimiothérapeutique en cancérologie atteint un plateau. En effet, il faut reconnaître que la chimiothérapie est en échec devant les tumeurs solides les plus fréquentes dans les sociétés occidentales : cancer du sein, du poumon, de la prostate, tumeurs digestives et urinaires... Elle permet souvent de diminuer le degré de gravité des tumeurs malignes, mais conduit rarement à la guérison.

US-A-5 093 505 décrit des dérivés de 4-pyranone utiles comme agents carcinostatiques.

L'un des premiers objectifs de la pharmacologie des médicaments anticancéreux est donc la recherche constante de nouvelles drogues susceptibles de manifester une meilleure efficacité thérapeutique. Les deux premiers critères mis en jeu dans la sélection de nouveaux médicaments antitumoraux sont :
1. l'originalité de la structure chimique et du mécanisme d'action,
2. l'activité antitumorale expérimentale dans des modèles cellulaires *in vitro* mais surtout dans des modèles animaux *in vivo.*

La Demanderesse a ainsi trouvé de nouveaux composés dérivés de pyranone présentant l'ensemble de ces critères.

La présente invention a pour objet des composés de formule générale (I) 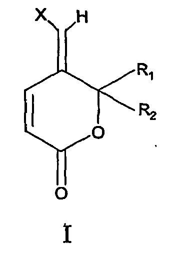 dans laquelle
X représente le chlore, le brome ou l'iode et
R₁ et R₂ représentent chacun indépendamment l'un de l'autre un atome d'hydrogène, un groupe alkyle, cycloalkyle ou alkylène, linéaire ou ramifié, avantageusement linéaire, contenant de 1 à 20 atomes de carbone, éventuellement substitué par un groupe hydroxyle, amino, éther ou halogène, ou R₁ et R₂ forment ensemble un cycle de 5, 6, 7 ou 8 membres, ledit cycle étant éventuellement substitué par un groupe hydroxyle, amino, éther ou halogène,
y compris ses isomères, ses énantiomères, ses diastéréoisomères, et leurs mélanges.

Les composés de formule (I) peuvent comporter un ou plusieurs atomes de carbone asymétriques quand R₁ et R₂ sont différents l'un de l'autre. Ils peuvent donc exister sous forme d'énantiomères ou de diastéréoisomères. Les composés de formule (I) peuvent également se présenter sous forme d'isomères cis ou trans. Ces isomères, énantiomères, diastéréoisomères, ainsi que leurs mélanges, y compris les mélanges racémiques font partie de l'invention.

Avantageusement selon la présente invention, R₁ et R₂ comprennent de 1 à 15 atomes de carbone, encore plus avantageusement de 1 à 10 atomes de carbone, encore plus avantageusement de 1 à 5 atomes de carbone.

Dans un mode de réalisation particulier selon la présente invention, R₁ et R₂ forment ensemble un cycle de 5, 6, 7 ou 8 membres, le cycle étant avantageusement un cycle hydrocarboné saturé. Dans un mode de réalisation particulier préféré selon l'invention, R₁ et R₂ forment ensemble un cycle hydrocarboné saturé de 5 ou 6 membres.

Dans le cadre de la présente invention, on entend par :
- un groupe alkyle, un groupe aliphatique saturé linéaire ou ramifié; on peut notamment citer les groupes méthyle, éthyle, propyle, isopropyle, butyle, isobutyle, tertiobutyle, pentyle, etc,
- un groupe cycloalkyle, un groupe alkyle cyclique ; on peut notamment citer les groupes cyclopropyle, cyclobutyle, cydopentyle, cyclohexyle, etc,
- un groupe halogène, un fluor, un chlore, un brome ou un iode,
- un groupe alkylène, un groupe aliphatique mono ou poly-insaturé, linéaire ou ramifié, comprenant de préférence une ou deux saturations éthyléniques,
- un groupe amino, un groupe NH₂ ou un groupement amine secondaire ou tertiaire,
- un groupe éther, un groupe OR', R' étant un radical alkyle, tel que le méthyle, éthyle, propyle, isopropyle, butyle, isobutyle, tertiobutyle, pentyle, etc.

Parmi les composé de formule (I) objets de la présente invention, on peut citer les composés préférés qui se définissent comme suit : X représente le chlore, le brome ou l'iode, et R₁ et R₂ représentent chacun indépendamment l'un de l'autre un atome d'hydrogène, un groupe alkyle ou alkylène, linéaire ou ramifié, avantageusement linéaire, contenant de 1 à 20 atomes de -carbone, éventuellement substitué par un groupe éther ou halogène, ou R₁ et R₂ forment ensemble un cycle de 5, 6, 7 ou 8 membres, ledit cycle étant éventuellement substitué par un groupe éther ou halogène.

Parmi ces derniers composés préférés, on préfère tout particulièrement les composés de formule (I) pour lesquels X représente l'iode.

Parmi ces derniers composés préférés, on préfère tout particulièrement les composés de formule (I) pour lesquels R₁ et R₂ représentent chacun indépendamment l'un de l'autre un atome d'hydrogène, un groupe méthyle, éthyle, propyle ou butyle.

Avantageusement selon la présente invention, R₁ et R₂ représentent chacun un groupe méthyle ou l'un de ces deux radicaux représente un groupe méthyle et l'autre représente un atome d'hydrogène.

Avantageusement selon la présente invention, X représente l'iode et R₁ et R₂ représentent chacun un groupe méthyle ou l'un de ces deux radicaux représente un groupe méthyle et l'autre représente un atome d'hydrogène.

Parmi ces derniers composés préférés, on préfère tout particulièrement les composés de formule (I) qui sont des iodométhylène-diméthyl-dihydropyranones, pour lesquels X représente l'iode et R₁ et R₂ représentent chacun un groupe méthyle.

De manière encore plus avantageuse selon la présente invention, le composé de formule (I) est l'isomère E-iodométhylène-diméthyl-dihydropyranone.

La présente invention a également pour objet un procédé de préparation des composés de formule générale (I), dans lequel on procède d'abord à une réaction de Horner-Emmons en faisant réagir un aldéhyde de formule (IV) 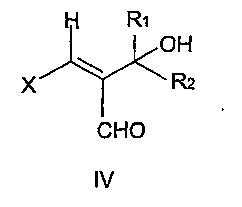 dans laquelle les significations de X, R₁, et R₂ sont celles définies ci-dessus pour les composés de formule (I),
avec un phosphonate tel que le [bis (2,2,2-trifluoroéthyle)-phosphinoyle]-acétate de méthyle, puis on procède à une cyclisation (lactonisation). D'autres phosphonates tels que le [bis (2,4-difluorophényle)-phosphinoyle]-acétate de méthyle, le diphényle phosphinoyle-acétate de méthyle, ou les phosphinoyle-acétate d'éthyle cycliques dérivés de la N,N'-diméthyl éthylènediamine décrits par : Carl Patois et Philippe Savignac Tetrahedron lett.,1991,32, 1317-1320, peuvent être utilisés dans le cadre de la présente invention.

Avantageusement selon la présente invention, la préparation du composé de formule (I) à partir du composé de formule (IV) est réalisée en présence d'une base, avantageusement faible, telle que le carbonate de potassium et d'un éther couronne tel que l'éther couronne 18-crown-6. D'autres bases telles que le KN(TMS)₂, le Triton B, le NaH, le LDA ou le 2,2,6,6-tétraméthyl pipéridine peuvent être utilisés dans le cadre de la présente invention. La présence de l'éther couronne permet de complexer le cation formé associé à la base et de favoriser ainsi l'obtention d'une oléfine de configuration Z requise pour la lactonisation. Cette étape consistant à passer du composé de formule (IV) au composé de formule (I) est de préférence réalisée dans un solvant anhydre du type toluène, sous atmosphère inerte.

Les composés de formule générale (I) peuvent ainsi être synthétisés selon le schéma 1 suivant :

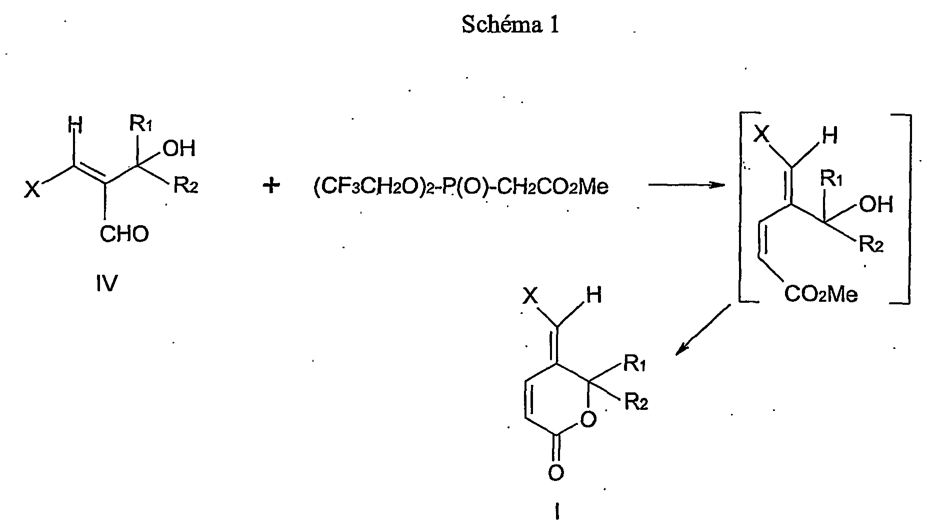

Dans un mode de réalisation particulier de la présente invention, la préparation du composé de formule (I) à partir du composé de formule (IV) est précédée des étapes suivantes :
i) on fait tout d'abord réagir un composé de formule (II) avec un agent réducteur tel que l'aluminohydrure de lithium pour conduire à la formation de l'alcool primaire correspondant (III), puis
ii) on fait réagir le composé de formule (III) avec un agent oxydant tel que le dioxyde de manganèse pour obtenir l'aldéhyde correspondant (IV) 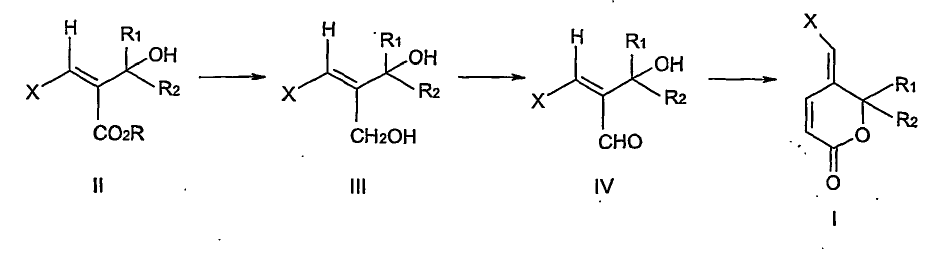 dans lesquelles les significations de X, R₁, et R₂ sont celles définies ci-dessus pour les composés de formule (I), et R représente un groupe alkyle linéaire contenant de 1 à 5 atomes de carbone, tel qu'un groupe méthyle ou éthyle.

Le composé (II) utilisé selon la présente invention est avantageusement sous forme de l'isomère cis (Z). Le composé (II) peut être synthétisé à partir de propiolate d'alkyle (HC≡C-CO₂R) et de cétone (R₁COR₂), R, R₁ et R₂ étant définis comme ci-dessus, en présence d'halogénure de tétrabutylammonium, d'un solvant anhydre du type chlorure de méthylène et d'un acide de Lewis tel que le tétrachlorure de zirconium ou l'iodure de diéthyl aluminium. La synthèse du composé (II) se fait de préférence aux environs de 0°C, sous atmosphère inerte.

L'étapé (i) du procédé selon la présente invention, consistant à réduire la fonction ester du composé (II) pour conduire à la formation de l'alcool primaire correspondant (III), est réalisée à l'aide d'un agent réducteur tel que l'aluminohydrure de lithium, le borohydrure de lithium ou l'hydrure de diisobutyl aluminium. Cette étape (i) est de préférence réalisée dans un solvant anhydre de type éther, sous atmosphère inerte, à température ambiante.

L'étape (ii) du procédé selon la présente invention, consistant à oxyder la fonction alcool primaire du composé (III) pour conduire à la formation de l'aldéhyde correspondant (IV), est réalisée à l'aide d'un agent oxydant tel que le dioxyde de manganèse ou le periodinane de Dess-Martin. Cette étape (ii) est de préférence réalisée dans un solvant anhydre du type chlorure de méthylène, sous atmosphère inerte, à température ambiante.

Dans un mode de réalisation particulier de la présente invention, lorsque les radicaux R₁ et R₂ des composés de formule générale (I) sont substitués par des groupements du type hydroxyle ou amino, lesdits groupements sont protégés tout au long de la synthèse pour passer des composés de formule (II) aux composés de formule (III) puis (IV), et des composés de formule (IV) aux composés de formule (I) par des groupes protecteurs.

On entend par groupe protecteur au sens de la présente invention, un groupe qui permet d'une part de protéger une fonction réactive telle qu'un hydroxy ou une amine pendant une synthèse et d'autre part de régénérer la fonction réactive intacte en fin de synthèse. Des exemples de groupes protecteurs ainsi que les méthodes de protection et déprotection sont données dans Protective groups in Organics Synthesis, Green et at., 2^{nd} Ed. (John Wiley & Sons, Inc., New York).

La présente invention a également pour objet les composés intermédiaires de formule générale (IV) 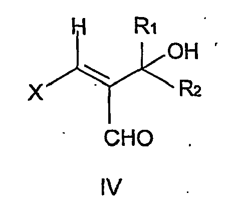 dans laquelle les significations de X, R₁, et R₂ sont celles définies ci-dessus pour les composés de formule (I),
y compris ses isomères, ses énantiomères, ses diastéréoisomères, et leurs mélanges.

Les composés de formule (IV) peuvent comporter un ou plusieurs atomes de carbone asymétriques quand R₁ et R₂ sont différents l'un de l'autre. Ils peuvent donc exister sous forme d'énantiomères ou de diastéréoisomères. Les composés de formule (I) correspondants peuvent se présenter majoritairement sous forme d'isomères cis ou trans, avantageusement sous forme d'isomères trans, notamment lorsque ni R₁ ni R₂ ne représentent l'atome d'hydrogène. Les composés de formule (I) peuvent également se présenter sous forme d'un mélange d'isomères cis et trans, en particulier lorsque R₁ ou R₂ représente l'atome d'hydrogène. Ces isomères, énantiomères, diastéréoisomères, ainsi que leurs mélanges, y compris les mélanges racémiques font partie de l'invention.

Avantageusement selon la présente invention, les radicaux R₁ et R₂ des composés (IV) comprennent de 1 à 15 atomes de carbone, encore plus avantageusement de 1 à 10 atomes de carbone, encore plus avantageusement de 1 à 5 atomes de carbone.

Parmi les composé de formule (IV) objets de la présente invention, on peut citer les composés préférés qui se définissent comme suit : X représente l'iode.

Avantageusement selon la présente invention, R₁ et R₂ représentent chacun un groupe méthyle ou l'un de ces deux radicaux représente un groupe méthyle et l'autre représente un atome d'hydrogène.

Avantageusement selon la présente invention, X représente l'iode et R₁ et R₂ représentent chacun un groupe méthyle ou l'un de ces deux radicaux représente un groupe méthyle et l'autre représente un atome d'hydrogène.

Parmi ces derniers composés préférés, on préfère tout particulièrement les composés de formule (IV), pour lesquels X représente l'iode et R₁ et R₂ représentent chacun un groupe méthyle.

Les composés (I) de l'invention ont fait l'objet d'essais pharmacologiques permettant de déterminer leur activité antitumorale et leur activité cytotoxique sur des lignées cellulaires cancéreuses.
1. Des essais ont consisté à mesurer l'activité cytotoxique *in vitro* des composés de l'invention sur des lignées cellulaires cancéreuses d'origines tissulaires différentes (leucémie, cancer du sein, du colon et de la bouche), ainsi que sur des cellules chimiorésistantes.
   Il apparaît ainsi que les composés (I) de l'invention bloquent la division des cellules tumorales en phase G2M du cycle cellulaire et entraînent la mort de ces cellules par apoptose.
2. D'autres essais consistant à mesurer l'activité antitumorale *in vivo* des composés (I) de l'invention on été effectués.

Cette activité antitumorale des composés de l'invention a été étudiée sur des tumeurs greffées (telles que HCT-116) chez des souris Nude (souris *nu*/*nu*). Les souris Nude sont des souris immunodéficitaires dépourvues de thymus.

Il apparaît donc que les composés (I) de l'invention ont une activité antitumorale, et peuvent donc être utilisés pour la préparation de médicaments présentant une activité antitumorale et une activité cytotoxique sur des lignées cellulaires cancéreuses. Ces médicaments trouvent leur emploi en thérapeutique, notamment dans le traitement du cancer.

Ainsi, un des objets de la présente invention est un médicament consistant en un composé de l'invention de formule (I).

Selon un autre de ses aspects, la présente invention concerne des compositions pharmaceutiques comprenant en tant que principe actif, un composé (I) selon l'invention. Ainsi, ces compositions pharmaceutiques contiennent une dose efficace d'un composé (I) selon l'invention, avec tout excipient approprié, notamment un ou plusieurs excipient(s) pharmaceutiquement acceptable(s). Lesdits excipients sont choisis selon la forme pharmaceutique et le mode d'administration souhaité.

Les compositions pharmaceutiques selon la présente invention sont avantageusement destinées à être administrées par injection intraveineuse. Les compositions pharmaceutiques selon la présente invention peuvent également être administrées par les voies d'administration suivantes : voie orale, sublinguale, sous-cutanée, intramusculaire, topique, intratrachéale, intranasale, transdermique ou rectale.

Le principe actif de formule (I) ci-dessus peut être administré sous forme unitaire d'administration, en mélange avec des excipients pharmaceutiques classiques, pour le traitement du cancer. Les formes unitaires d'administration appropriées comprennent les formes par voie orale, telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades ou lotions.

Lorsqu'on prépare une composition solide sous forme de comprimés, on mélange l'ingrédient actif principal avec un excipient pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières. Les comprimés peuvent être réalisés par différentes techniques, compression directe, granulation sèche, granulation humide ou fusion à chaud.

On obtient une préparation en gélules en mélangeant l'ingrédient actif avec un diluant et en versant le mélange obtenu dans des gélules molles ou dures.

Pour une administration parentérale, on peut utiliser des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

La présente invention, selon un autre de ses aspects, concerne également l'utilisation d'un composé de formule (I) selon l'invention pour la préparation d'un médicament destiné à traiter le cancer.

Les exemples suivants illustrent la présente invention.

### Exemple 1 : Préparation de composés de formule (I) : E-5-iodométhylène-6,6-diméthyl-5,6-dihydro-pyran-2-one

### X = I, R₁ = CH₃, et R₂ = CH₃

La synthèse de (I) a été réalisée à partir du Z-2-(1-hydroxy-1-méthyl-éthyl)-3-iodo-acrylate d'éthyle (II) selon les procédés décrits respectivement par Villieras et coll (Taïcir Ben Ayed, Jean Villieras, Hassan Ari, Tetrahedron, 2000, 56, 805-809) ou par P.W. Paré et coll (Han-Xun Wei, Joe J. Gao, Guigen Li, Paul W. Paré, Tetrahedron Lett., 2002, 43, 5677-5680). La réduction de la fonction ester de (II) par l'aluminohydrure de lithium fournit l'alcool primaire (III). L'oxydation de la fonction alcool primaire par le dioxyde de manganèse ou par le periodinane de Dess-Martin fournit l'aldéhyde (IV). Le couplage de (IV) avec le [bis (2,2,2-trifluoroéthyle)-phosphinoyle]-acétate de méthyle en utilisant le carbonate de potassium comme base en présence d'éther couronne 18-crown-6 conduit à l'iodure vinylique désiré (I) avec un rendement de 50% à partir du composé (II) (schéma 2) 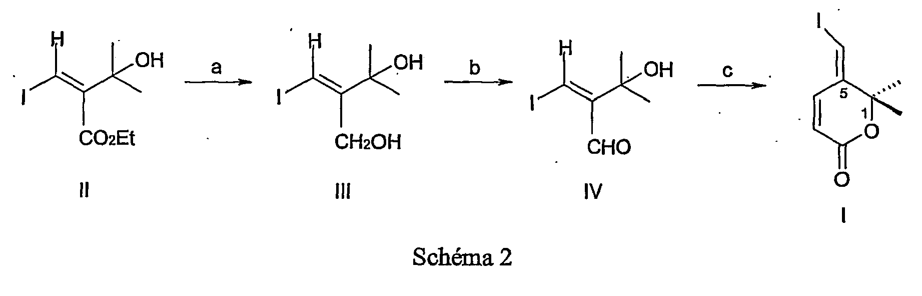 **Schéma 2** *conditions réactionnelles:* a- LAH, 1 éq., éther, température ambiante (ta), 1h, 75% ; b- MnO₂, 10 éq., température ambiante, CH₂Cl₂, 3h, 90% ; c- K₂CO₃, 6 éq., 18-crown-6/CH₃CN, 12 éq., toluène, 25°C, 1h, puis -20°C, **(IV)** 1 éq. et (CF₃CH₂O)₂-P(O)-CH₂CO₂Me, 1 éq., - 20°C à 0°C, puis 30 min à 0°C, 74%.

### 1.1. Préparation de composés de formule (II): Z-2-(1-hydroxy-1-méthyl-éthyl)-3-iodo-acrylate d'éthyle

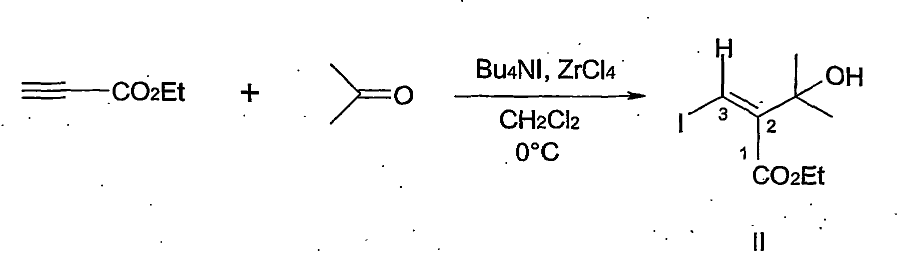

A un mélange de propiolate d'éthyle (19,3 mmoles, 1,9 g ou 1,96 mL), d'acétone redistillée sur sulfate de calcium (24 mmoles, 1,76 mL) et d'iodure de tétrabutyl ammonium (21,6 mmoles, 8 g) en solution dans 100 mL de chlorure de méthylène anhydre sous atmosphère inerte (argon), on ajoute à 0°C du tétrachlorure de zirconium (24 mmoles, 5,6 g). La solution est agitée à 0°C sous Argon pendant 5 h. Après addition d'eau (20 mL), les produits organiques sont extraits par du chlorure de méthylène (3 fois 50 mL). Les phases organiques séchées (MgSO₄) sont évaporées sous pression réduite pour fournir un résidu (8,3 g) qui est chromatographié sur gel de silice 60H Merck. Par élution avec un mélange CH₂Cl₂/MeOH 98/2 puis 97/3, on obtient le composé **(II)** (1,28 g, 23%).
¹H RMN : δ ppm (CDCl₃, 250 MHz) 1,32 (3H, t, J=7,1 Hz, CH₃-CH₂), 1,39 (6H, s, (CH₃)₂-C), 2,83 (1H, s, OH), 4,27 (2H, q, J=7,1 Hz, CH₃-CH₂), 6,77 (1H, s, H-3).

### Méthode alternative de préparation des composés de formule (II) :

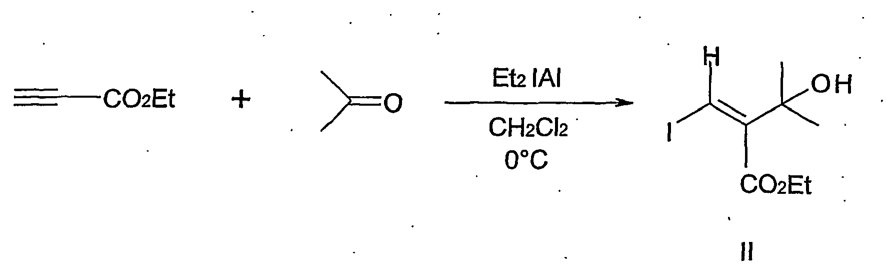

A un mélange de propiolate d'éthyle (13 mmoles, 1,31 mL), d'acétone redistillée sur sulfate de calcium (10 mmoles, 0,733 mL), en solution dans 50 mL de chlorure de méthylène anhydre, sous atmosphère inerte (argon) maintenue à 0°C sous agitation, on ajoute en 30 minutes une solution d'iodure de diéthyl aluminium dans le toluène (12 mmoles, 12 mL). La solution jaune obtenue est agitée 2h, à 0°C. La réaction est quenchée par addition lente, à 0°C, d'une solution d'acide chlorhydrique 2N.

Après addition d'eau et décantation, les produits organiques sont extraits par de l'acétate d'éthyle (3 fois 50 mL). Les phrases organiques sont lavées par une solution saturée de NaCl, séchées sur MgSO₄, concentrées sous pression réduite pour fournir une huile jaune (2,04 g, 69%), dont le spectre de RMN est identique à celui du composé II obtenu par la première méthode de préparation mentionnée ci-déssus.

### 1.2. Préparation de composés de formule (III) : E-2-(1-hydroxy-1-méthyl-éthyl)-3-iodo-prop-2-èn-1-ol

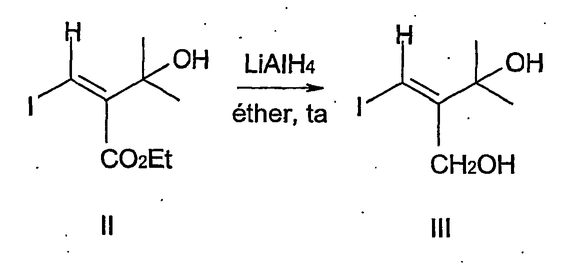

A une solution de Z-2-(1-hydroxy-1-méthyl-éthyl)-3-iodo-acrylate d'éthyle (II) (4,9 mmoles, 1,4 g) dans l'éther anhydre (30 mL) on ajoute de l'aluminohydrure de lithium (2,9 mmoles, 0,112 g). La réaction est agitée 1 h sous atmosphère inerte (argon) à la température ambiante (ta). Après destruction de l'excès d'aluminohydrure de lithium par addition d'une solution saturée de sulfate de sodium (30 µl), le précipité d'alumine est filtré. Le filtrat séché (MgSO₄), et évaporé sous pression réduite, fournit un résidu blanc de E-2-(1-hydroxy-1-méthyl-éthyl)-3-iodo-prop-2-èn-1-ol **(III)** (0,9 g, 75%).
¹HRMN : δ ppm, CDCl₃, 250 MHz: 1,42 (6H, s, (CH₃)₂-C), 4,44 (2H, s, CH₂OH), 6,48 (1H, s, H-3).

### 1.3. Préparation de composés de formule (IV) : Z-2-(1-hydroxy-1-méthyl-éthyl)-3-iodo-propénal

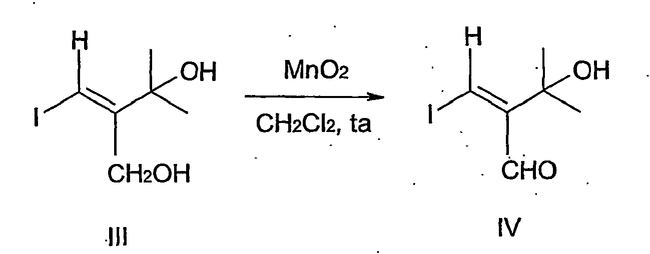

A une solution de E-2-(1-hydroxy-1-métlhyl-éthyl)-3-iodo-prop-2-èn-1-ol (III) (5,7 mmoles, 1,4 g) dans le chlorure de méthylène sec (25 mL), on ajoute par portions du dioxyde de manganèse (10 éq., 57 mmoles, 5 g), à la température ambiante (ta), sous atmosphère inerte. L'évolution de la réaction est suivie par chromatographie analytique sur plaque de gel de silice (éluant CH₂Cl₂/MeOH 97/3). Après disparition du produit de départ, le mélange est filtré sur Célite®. Le filtrat évaporé sous pression réduite fournit le Z-2-(1-hydroxy-1-méthyl-éthyl)-3-iodo-propénal **(IV)** (1,3 g, 94%) sous forme d'une huile légèrement jaune.
SM ES M m/z 307 (M+44), 295 (M+MeOH), 263 (M+Na).
S.M.H.R (SM ES+) : calculée pour C₆H₉O₂NaI : 262,9545 ; mesurée :262,9545
¹H RMN : δ ppm, CDCl₃, 250 MHz : 1,43 (6H, s, (CH₃)₂-C), 7,97 (1H, s, H-3), 9,79 (1H, s, CHO).
¹³C RMN : δ ppm, CDCl₃, 28,7 ((CH₃)₂-C), 74,2 (C-OH), 101,8 (C3-I), 149,9 (=C2), 196,8 (CHO).
IR υ : 1681 cm⁻¹ (C=O conjugué), 1565 cm⁻¹ (C=C), 1369, 1282, 1176, 1085 et 963 cm⁻¹.

### 1.4. Préparation de composés de formule (I): E-5-iodométhylène-6,6-diméthyl-5,6-dihydro-pyran-2-one

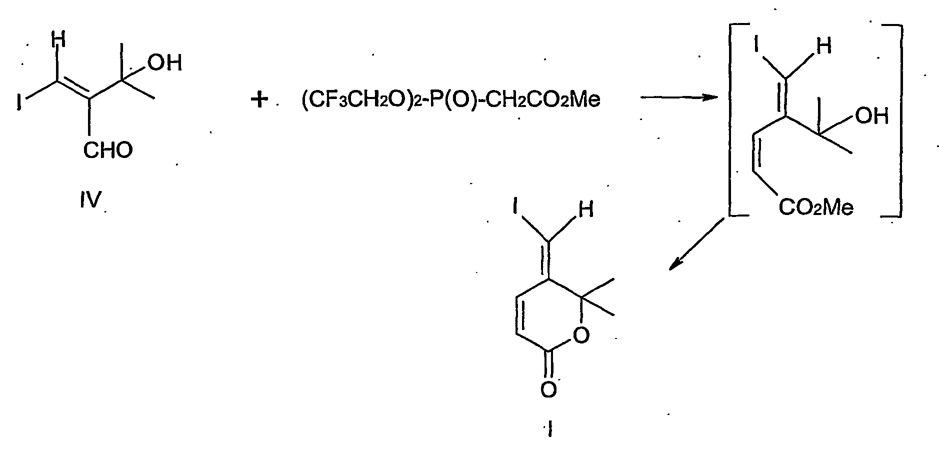

### 1.4.1. Purification de l'éther couronne 18-crown-6

Dans un ballon de 500 mL, 25 g d'éther couronne 18-crown-6 commercial et 63 ml d'acétonitrile sec sont chauffés et agités jusqu'à complète dissolution, à l'abri de l'humidité. On laisse refroidir à température ambiante, puis on plonge le ballon dans un bain de glace/acétone. Les cristaux blancs du complexe, se déposent et sont recueillis par filtration. Ces cristaux hygroscopiques sont transférés dans un ballon de 250 mL muni d'un barreau magnétique. L'acétonitrile est évaporé sous vide poussé (0,1-0,5 Torr), à une température ≤40°C, pendant 2 à 3 h. On obtient 25 g d'éther couronne/CH₃CN.

### 1.4.2. Préparation de (I)

Un mélange de carbonate de potassium (32 mmoles, 4,48 g) et d'éther couronne 18-crown-6/CH₃CN (64,8 mmoles, 17,1 g) dans du toluène anhydre (54 mL) est agité sous atmosphère inerte, 1h à 25°C et la solution légèrement trouble obtenue est refroidie à -20°C. Le Z-2-(1-hydroxy-1-méthyl-éthyl)-3-iodo-propénal (IV) (5,4 mmoles, 1,3 g) et le bis-trifluoroéthyl-phosphono-acétate de méthyle (5,4 mmoles, 1,72 g ou 1,14 mL) sont ajoutés et le mélange obtenu est agité et laissé réchauffer jusqu'à 0°C. Le mélange devient blanc crémeux. Après agitation 30 minutes à 0°C sous atmosphère inerte, une solution de chlorure d'ammonium est ajoutée pour quencher la réaction et les produits organiques sont alors extraits par de l'éther. Les phases organiques séchées sur MgSO₄ sont évaporées sous pression réduite et fournissent un produit cristallisé jaune-vert (1,5 g). Par chromatographie sur colonne de gel de silice 60H, effectué avec un gradient d'heptane/éther, on obtient la E-6-diméthyl-5-iodométhylène-5,6-dihydro-pyran-2-one **(I)** cristallisée, légèrement colorée (1,06 g, 74%).
SM ES m/z 287 (M+Na)
S.M.H.R. (SM ES+) : calculée pour C₈H₉O₂Nal :286,9545 ; mesurée : 286,9530.
¹H RMN : δ ppm, CDCl₃, 250 MHz : 1,62 (6H, s, (CH₃)₂-C), 6,1 (1H, dd, J=10, J'=2 Hz, H-4), 6,88 (1H, d, J=2 Hz, H-9), 7,2 (1H, d, J=10 Hz, H-3), nOe entre H-9 et CH₃.
¹³C RMN : δ ppm, CDCl₃, 28,9 ((CH₃)₂-C), 84,0 (C6-O), 86,4 (C9-I), 121,6 (=C4H), 142,3 (C3H=), 144,9 (C5), 163,3 (C=O).
IR υ : 1695 cm⁻¹ (C=O conjugué), 1560, 1394, 1290, 1176,-1115, 1089 et 990 cm⁻¹.
IESM : 264 M⁺, m/z 137, 127.
UV (EtOH) λ nm : 300, log ∈ 4,1.

### Exemple 2 : Etude biologique in vitro des composés (I) selon l'invention

L'activité biologique de l'iodométhylène-diméthyl-dihydropyranone (I), tel qu'obtenu selon l'exemple 1, a été étudiée *in vitro* sur cinq lignées cellulaires différentes:
- KB (carcinome épidermoïde du nasopharynx)
- HCT-116 (carcinome colorectal)
- K562 (leucémie myéloïde)
- K562-MDR1 (leucémie myéloïde; résistance à la doxorubicine)
- MCF7-MDR1 (adénocarcinome mammaire; résistance à la doxorubicine)

Les cellules sélectionnées pour cette étude ont été incubées à 37°C en présence de l'iodométhylène-diméthyl-dihydropyranone (I) ajoutée dans le milieu de culture à différentes concentrations. L'ensemble des expériences réalisées a permis de déterminer le degré de toxicité du composé testé, son effet sur le déroulement du cycle cellulaire ainsi que sa capacité à induire une mort cellulaire par apoptose.

### 2.1. Etude de la cytotoxicité

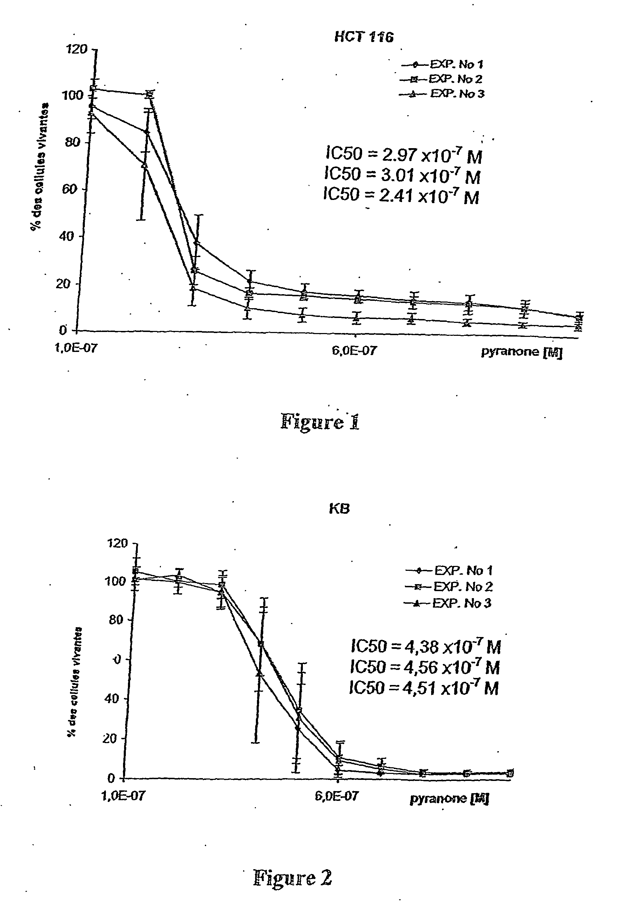

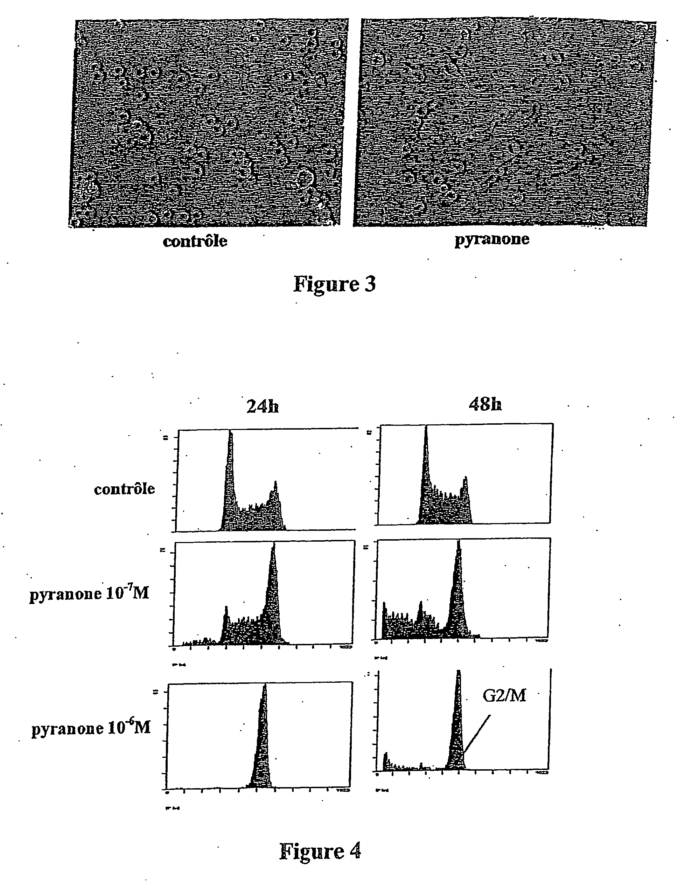

La cytotoxicité de l'iodométhylène-diméthyl-dihydropyranone (I) a été évaluée vis-à-vis des cellules KB et HCT-116. La concentration de l'iodométhylène-diméthyl-dihydropyranone qui induit la mort de 50 % des cellules (IC50) a été déterminée après 96 heures d'incubation et elle est de l'ordre de 0,30 micromolaire pour les cellules HCT-116 (Figure 1) et 0,45 micromolaire pour les cellules KB (Figure 2).
Il faut également souligner que dès 24 heures après le traitement avec l'iodométhylène-diméthyl-dihydropyranone (I) à la dose 10⁻⁷M, on observe un changement de la morphologie de cellules traitées. En effet, les cellules perdent leurs formes arrondies et deviennent fusiformes (Cf. Figure 3 : traitement de cellules K562 pendant 24 heures par le composé (I) à la dose 10⁻⁷M).

### 2.2. Etude du cycle cellulaire

L'analyse par cytométrie en flux des cellules traitées (K562, K562-MDR1, HCT116, MCF7-MDR1) avec l'iodométhylène-diméthyl-dihydropyranone (I) a montré que ce composé bloque la division cellulaire de toutes les lignées en phase G2/M. Cet effet est significatif après 24 heures d'exposition des cellules à l'iodométhylène-diméthyl-dihydropyranone (I) utilisée à la concentration 10⁻⁷ M (Cf. Figure 4 : effet de (I) sur le cycle cellulaire des cellules leucémiques K562).

### 2.3. Etude de l'apoptose

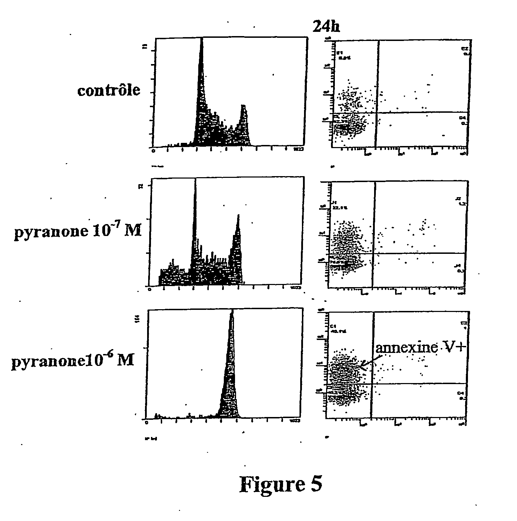

Afin de préciser si l'iodométhylène-diméthyl-dihydropyranone (I) entraîne une mort cellulaire par apoptose, les cellules K562 traitées pendant 24 heures ont été analysées par cytométrie en flux en utilisant le double marquage : Annexine V et Iodure de Propidium (IP). Les résultats présentés dans la Figure 5 montrent que l'incubation des cellules K562-MDR1 (chimiorésistantes) pendant 24 heures avec l'iodométhylène-diméthyl-dihydropyranone (I) aux concentrations 10⁻⁶ M et 10⁻⁷ M conduit à une forte induction de l'apoptose (cellules annexine V positives / IP négatives).

### Exemple 3 : Etude biologique in vivo des composés (I) selon l'invention

### 3.1. Détermination de la dose maximale tolérée de l'iodométhylène-diméthyl-dihydropyranone (I) pour une injection unique (DMT) et pour quatre injections répétées (DMTT)

L'iodométhylène-diméthyl-dihydropyranone (I), tel qu'obtenu selon l'exemple 1, a été injectée par voie intraveineuse (iv) à des souris Nude (Swiss Nu/nu) âgées de 4-5 semaines aux doses: 50 mg/kg, 66,6 mg/kg, 100 mg/kg, 150 mg/kg et 200 mg/kg. Le suivi de la survie des animaux, ainsi que de leur poids, jusqu'au jour 21 a révélé une toxicité pour les doses de 150 et 200 mg/kg, ce qui situe la DMT à 100 mg/kg.
La DMT déterminée, les souris Nude ont été traitées par voie intraveineuse (iv) avec l'iodométhylène-diméthyl-dihydropyranone (I) injectée aux trois doses différentes : 50, 70 et 90 mg/kg/injection. Chaque dose a été administrée 4 fois à 3 jours d'intervalle (J0, J3, J6 et J9). La survie des animaux, leur poids et des signes cliniques notés jusqu'au jour 21 ont permis d'établir la valeur de DMTT qui se situe au dessous de 90 mg/kg. Les doses 40, 60 et 80 mg/kg ont donc été choisies pour l'évaluation de l'activité antitumorale.

### 3.2. Evaluation de l'activité antitumorale de l'iodométhylène-diméthyl-dihydropyranone (I) administrée par voie intraveineuse chez la souris Nude porteuse de tumeur greffée HCT 116

La xénogreffe de cellules tumorales humaines chez la souris Nude est un modèle couramment utilisé pour l'évaluation de l'activité antitumorale de diverses molécules.

Les souris Nude porteuses d'une tumeur HCT 116 greffée sous la peau ont été traitées avec l'iodométhylène-diméthyl-dihydropyranone (I) administrée par voie intraveineuse (iv) à trois doses différentes (40, 60 et 80 mg/kg/injection), choisies en fonction de la tolérance à ce composé démontrée dans l'étude précédente. L'iodométhylène-diméthyl-dihydropyranone (I) a été injectée 4 fois à 3 jours d'intervalle (J0, J3, J6 et J9). Deux groupes d'animaux contrôles ont été constitués. Le premier groupe n'a reçu aucune injection (témoin), alors que les animaux du second groupe ont reçu des injections de la solution qui a servi à solubiliser l'iodométhylène-diméthyl-dihydropyranone (I) (véhicule).

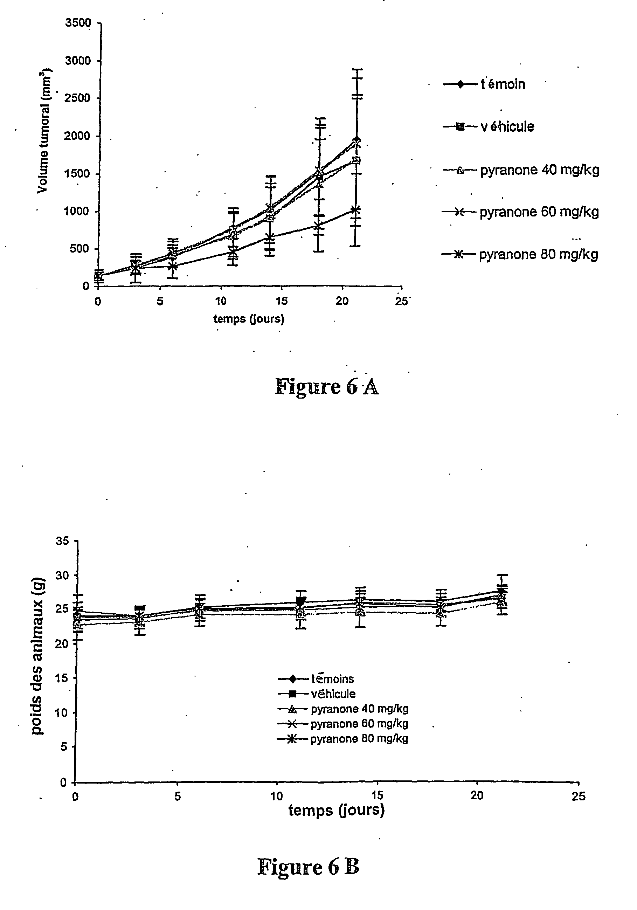

Le volume des tumeurs (mm³) a été mesuré avec un pied à coulisse deux fois par semaine. Les courbes de la croissance tumorale (Figure 6A) montrent qu'à partir de la 2^{ème} injection, l'iodométhylène-diméthyl-dihydropyranone (I) à la dose de 80 mg/kg/injection inhibe significativement la progression tumorale.

En se basant sur l'un des paramètres d'évaluation de l'inhibition de la croissance tumorale qu'est le pourcentage T/C [T/C = (médiane du volume des tumeurs dans le groupe traité / médiane du volume des tumeurs dans le groupe contrôle) x 100] qui atteint 53,5% 21 jours après le début des traitements, l'iodométhylène-diméthyl-dihydropyranone (1) présente une activité antitumorale proche de celle observée avec les drogues antitumorales efficaces selon les standards du NIH (T/C≤42%).

Les données présentées sur la Figure 6B montrent l'absence de la toxicité aiguë de l'iodométhylène-diméthyl-dihydropyranone (I), ce qui se traduit par une croissance normale des souris. En effet, le poids corporel des souris traitées augmente de façon homogène jusqu'à la fin du traitement.

## Revendications

1. Composé répondant à la formule (I) : 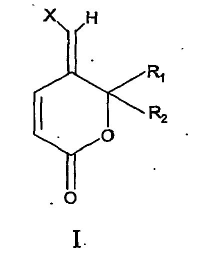 dans laquelle
X représente le chlore, le brome ou l'iode et
R₁ et R₂ représentent chacun indépendamment l'un de l'autre un atome d'hydrogène, un groupe alkyle, cycloalkyle ou alkylène, linéaire ou ramifié, contenant de 1 à 20 atomes de carbone, éventuellement substitué par un groupe hydroxyle, amino, éther ou halogène, ou R₁ et R₂ forment ensemble un cycle de 5, 6, 7 ou 8 membres, ledit cycle étant éventuellement substitué par un groupe hydroxyle, amino, éther ou halogène,
y compris ses isomères, ses énantiomères, ses diastéréoisomères, et leurs mélanges.

2. Composé dé formule (I) selon la revendication 1, **caractérisé en ce que** X représente le chlore, le brome ou l'iode, et R₁ et R₂ représentent chacun indépendamment l'un de l'autre un atome d'hydrogène, un groupe alkyle ou alkylène, linéaire ou ramifié, contenant de 1 à 20 atomes de carbone, éventuellement substitué par un groupe éther ou halogène, ou R₁ et R₂ forment ensemble un cycle de 5, 6, 7 ou 8 membres, ledit cycle étant éventuellement substitué par un groupe éther ou halogène.

3. Composé de formule (I) selon la revendication 1 ou 2, **caractérisé en ce que** X représente l'iode.

4. Composé de formule (I) selon l'une quelconque des revendications 1 à 3, **caractérisé en ce que** R₁ et R₂ représentent chacun indépendamment l'un de l'autre un atome d'hydrogène, un groupe méthyle, éthyle, propyle ou butyle.

5. Composé de formule (I) selon l'une quelconque des revendications 1 à 4, **caractérisé en ce que** R₁ et R₂ représentent chacun un groupe méthyle.

6. Composé de formule (I) selon l'une quelconque des revendications 1 à 5,
**caractérisé en ce qu'**il s'agit du iodométhylène-diméthyl-dihydropyranone.

7. Composé de formule (I) selon l'une quelconque des revendications 1 à 6, **caractérisé en ce qu'**il s'agit de l'isomère E-iodométhylène-diméthyl-dihydropyranone.

8. Procédé de préparation d'un composé de formule (I) selon l'une quelconque des revendications 1 à 7, **caractérisé en ce que** l'on procède à une réaction de Horner-Emmons en faisant réagir un aldéhyde de formule (IV) 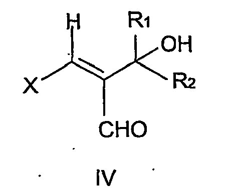 dans laquelle les significations de X, R₁, et R₂ sont celles définies pour le composé de formule (I) selon la revendication 1 ou 2,
avec un phosphonate tel que le [bis (2,2,2-trifluoroéthyle)-phosphinoyle]-acétate de méthyle, suivie d'une cyclisation.

9. Procédé selon la revendication 8, **caractérisé en ce que** la préparation du composé de formule (I) est réalisée en présence d'une base telle que le carbonate de potassium et d'un éther couronne tel que l'éther couronne 18-crown-6.

10. Procédé selon la revendication 8 ou 9, **caractérisé en ce que** la préparation du composé de formule (I) à partir du composé de formule (IV) est précédée des étapes suivantes :
i) on fait tout d'abord réagir un composé de formule (II) avec un agent réducteur tel que l'aluminohydrure de lithium pour conduire à la formation de l'alcool primaire correspondant (III), puis
ii) on fait réagir le composé de formule (III) avec un agent oxydant tel que le dioxyde de manganèse pour obtenir l'aldéhyde correspondant (IV) 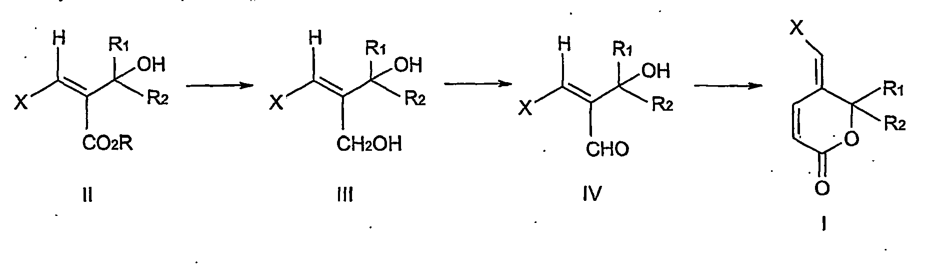
dans lesquelles les significations de X, R₁, et R₂ sont celles définies pour le composé de formule (I) selon la revendication 1 ou 2, et R représente un groupe alkyle linéaire contenant de 1 à 5 atomes de carbone, tel qu'un groupe méthyle ou éthyle.

11. Composé répondant à la formule (IV) : 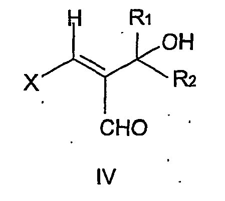 dans laquelle les significations de X, R₁, et R₂ sont celles définies pour le composé de formule (I) selon la revendication 1 ou 2,
y compris ses isomères, ses énantiomères, ses diastéréoisomères, et leurs mélanges.

12. Médicament **caractérisé en ce qu'**il consiste en un composé de formule (I) selon l'une quelconque des revendications 1 à 7.

13. Composition pharmaceutique **caractérisée en ce qu'**elle comprend un composé de formule (I) selon l'une quelconque des revendications 1 à 7, en association avec tout excipient approprié.

14. Composition selon la revendication 13, **caractérisée en ce qu'**elle est destinée à être administrée par injection intraveineuse.

15. Utilisation d'un composé de formule (I) selon l'une quelconque des revendications 1 à 7 pour la préparation d'un médicament destiné à traiter le cancer.

## Claims

1. Compound corresponding to formula (I): 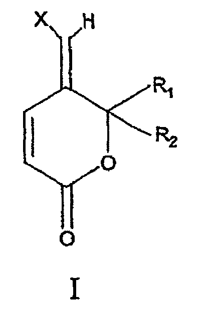 in which
X represents chlorine, bromine or iodine, and
R₁ and R₂ represent, each independently of the other, a hydrogen atom, an alkyl, cycloalkyl or alkylene group, which is linear or branched, containing from 1 to 20 carbon atoms, optionally substituted with a hydroxyl, amino, ether or halogen group, or R₁ and R₂ form together a 5-, 6-, 7- or 8-membered ring, said ring being optionally substituted with a hydroxyl, amino, ether or halogen group,
including its isomers, its enantiomers, its diastereoisomers, and mixtures thereof.

2. Compound of formula (I) according to Claim 1, **characterized in that** X represents chlorine, bromine or iodine, and R₁ and R₂ represent, each independently of the other, a hydrogen atom, an alkyl or alkylene group, which is linear or branched, containing from 1 to 20 carbon atoms, optionally substituted with an ether or halogen group, or R₁ and R₂ form together a 5-, 6-, 7-or 8-membered ring, said ring being optionally substituted with an ether or halogen group.

3. Compound of formula (I) according to Claim 1 or 2, **characterized in that** X represents iodine.

4. Compound of formula (I) according to any one of Claims 1 to 3, **characterized in that** R₁ and R₂ each represent independently of each other a hydrogen atom, a methyl, ethyl, propyl or butyl group.

5. Compound of formula (I) according to any one of Claims 1 to 4, **characterized in that** R₁ and R₂ each represent a methyl group.

6. Compound of formula (I) according to any one of Claims 1 to 5, **characterized in that** it is iodomethylene-dimethyl-dihydropyranone.

7. Compound of formula (I) according to any one of Claims 1 to 6, **characterized in that** it is the isomer E-iodomethylene-dimethyl-dihydropyranone.

8. Method for preparing a compound of formula (I) according to any one of Claims 1 to 7, **characterized in that** a Horner-Emmons reaction is carried out by reacting an aldehyde of formula (IV) 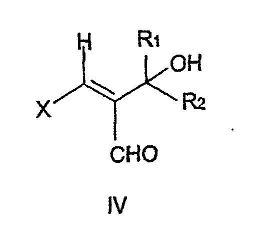 in which the meanings of X, R₁ and R₂ are those defined for the compound of formula (I) according to Claim 1 or 2, with a phosphonate such as methyl [bis(2,2,2-trifluoroethyl)phosphinoyl]acetate, followed by cyclization.

9. Method according to Claim 8, **characterized in that** the preparation of the compound of formula (I) is carried out in the presence of a base such as potassium carbonate and a crown ether such as the crown ether 18-crown-6.

10. Method according to Claim 8 or 9, **characterized in that** the preparation of the compound of formula (I) from the compound of formula (IV) is preceded by the following steps:
i) a compound of formula (II) is first of all reacted with a reducing agent such as lithium aluminum hydride, resulting in the formation of the corresponding primary alcohol (III), and then
ii) the compound of formula (III) is reacted with an oxidizing agent such as manganese dioxide to give the corresponding aldehyde (IV) 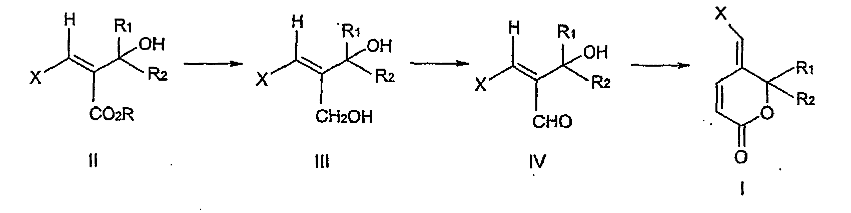
in which the meanings of X, R₁ and R₂ are those defined above for the compound of formula (I) according to Claim 1 or 2, and R represents a linear alkyl group containing from 1 to 5 carbon atoms, such as a methyl or ethyl group.

11. Compound corresponding to formula (IV): 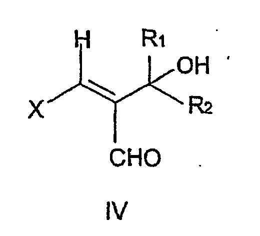 in which the meanings of X, R₁ and R₂ are those defined for the compound of formula (I) according to Claim 1 or 2,
including its isomers, its enantiomers, its diastereoisomers, and mixtures thereof.

12. Medicament, **characterized in that** it consists of a compound of formula (I) according to any one of Claims 1 to 7.

13. Pharmaceutical composition, **characterized in that** it comprises a compound of formula (I) according to any one of Claims 1 to 7, in combination with any appropriate excipient.

14. Composition according to Claim 13, **characterized in that** it is intended to be administered by intravenous injection.

15. Use of a compound of formula (I) according to any one of Claims 1 to 7, for the preparation of a medicament intended for treating cancer.

## Patentansprüche

1. Verbindung, die der Formel (I) entspricht: 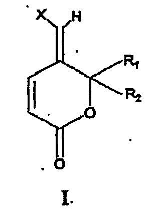 in der
X für Chlor, Brom oder Iod steht und
R₁ und R₂ jeweils unabhängig voneinander ein Wasserstoffatom, eine lineare oder verzweigte Alkyl-, Cycloalkyl- oder Alkylengruppe darstellen, die 1 bis 20 Kohlenstoffatome enthält und gegebenenfalls mit einer Hydroxyl-, Amino-, Ether- oder Halogengruppe substituiert ist, oder R₁ und R₂ zusammen einen Cyclus mit 5, 6, 7 oder 8 Gliedern bilden, wobei der Cyclus gegebenenfalls mit einer Hydroxyl-, Amino-, Ether- oder Halogengruppe substituiert ist, einschließlich ihrer Isomere, ihrer Enantiomere, ihrer Diastereoisomere und deren Mischungen.

2. Verbindung der Formel (I) nach Anspruch 1, **dadurch gekennzeichnet, dass** X für Chlor, Brom oder Iod steht und R₁ und R₂ jeweils unabhängig voneinander ein Wasserstoffatom, eine lineare oder verzweigte Alkyl- oder Alkylengruppe darstellen, die 1 bis 20 Kohlenstoffatome enthält und gegebenenfalls mit einer Ether- oder Halogengruppe substituiert ist, oder R₁ und R₂ zusammen einen Cyclus mit 5, 6, 7 oder 8 Gliedern bilden, wobei der Cyclus gegebenenfalls mit einer Ether- oder Halogengruppe substituiert ist.

3. Verbindung der Formel (I) nach Anspruch 1 oder 2, **dadurch gekennzeichnet, dass** X für Iod steht.

4. Verbindung der Formel (I) nach irgendeinem der Ansprüche 1 bis 3, **dadurch gekennzeichnet, dass** R₁ und R₂ jeweils unabhängig voneinander ein Wasserstoffatom, eine Methyl-, Ethyl-, Propyl- oder Butylgruppe darstellen.

5. Verbindung der Formel (I) nach irgendeinem der Ansprüche 1 bis 4, **dadurch gekennzeichnet, dass** R₁ und R₂ jeweils eine Methylgruppe darstellen.

6. Verbindung der Formel (I) nach irgendeinem der Ansprüche 1 bis 5, **dadurch gekennzeichnet, dass** es sich um lodmethylendimethyldihydropyranon handelt.

7. Verbindung der Formel (I) nach irgendeinem der Ansprüche 1 bis 6, **dadurch gekennzeichnet, dass** es sich um das Isomer E-lodmethylendimethyldihydropyranon handelt.

8. Verfahren zur Herstellung einer Verbindung der Formel (I) nach irgendeinem der Ansprüche 1 bis 7, **dadurch gekennzeichnet, dass** man eine Horner-Emmons-Reaktion durchführt, indem man einen Aldehyd der Formel (IV) 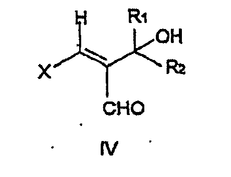 in der die Bedeutungen von X, R₁ und R₂ jene sind, die für die Verbindung der Formel (I) nach Anspruch 1 oder 2 definiert sind,
mit einem Phosphonat, wie Methyl[bis(2,2,2-trifluorethyl)phosphinoyl]acetat reagieren lässt, gefolgt von einer Cyclisierung.

9. Verfahren nach Anspruch 8, **dadurch gekennzeichnet, dass** die Herstellung der Verbindung der Formel (I) in Anwesenheit einer Base, wie Kaliumcarbonat oder eines Kronenethers, wie des Kronenethers 18-Krone-6, durchgeführt wird.

10. Verfahren nach Anspruch 8 oder 9, **dadurch gekennzeichnet, dass** der Herstellung der Verbindung der Formel (I) aus der Verbindung der Formel (IV) die folgenden Schritte vorangestellt sind:
i) man lässt zuerst eine Verbindung der Formel (II) mit einem Reduktionsmittel, wie Lithiumaluminiumhydrid, reagieren, was zur Bildung des entsprechenden primären Alkohols (III) führt, dann
ii) lässt man die Verbindung der Formel (III) mit einem Oxidationsmittel, wie Mangandioxid, reagieren, um den entsprechenden Aldehyd (IV) zu erhalten 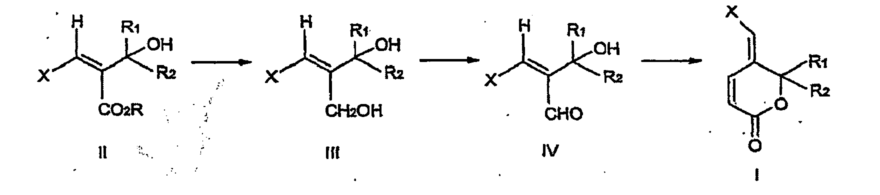
worin die Bedeutungen von X, R₁ und R₂ jene sind, die für die Verbindung der Formel (I) nach Anspruch 1 oder 2 definiert sind, und R eine lineare Alkylgruppe darstellt, die 1 bis 5 Kohlenstoffatome enthält, wie eine Methyl- oder Ethylgruppe.

11. Verbindung, die der Formel (IV) entspricht: 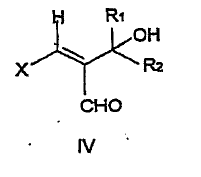 in der die Bedeutungen von X, R₁ und R₂ jene sind, die für die Verbindung der Formel (I) nach Anspruch 1 oder 2 definiert sind,
einschließlich ihrer Isomere, ihrer Enantiomere, ihrer Diastereoisomere und deren Mischungen.

12. Medikament, **dadurch gekennzeichnet, dass** es aus einer Verbindung der Formel (I) nach irgendeinem der Ansprüche 1 bis 7 besteht.

13. Pharmazeutische Zusammensetzung, **dadurch gekennzeichnet, dass** sie eine Verbindung der Formel (I) nach irgendeinem der Ansprüche 1 bis 7 in Kombination mit irgendeinem geeigneten Hilfsstoff enthält.

14. Zusammensetzung nach Anspruch 13, **dadurch gekennzeichnet, dass** sie dazu bestimmt ist, durch intravenöse Injektion verabreicht zu werden.

15. Verwendung einer Verbindung der Formel (I) nach irgendeinem der Ansprüche 1 bis 7 für die Herstellung eines Medikaments, das zur Behandlung von Krebs bestimmt ist.
